# EUROPEAN PATENT APPLICATION

(11) **EP 4 154 895 A2**
(43) Date of publication of application: **29.03.2023**
(21) Application number: 21807518.2
(22) Date of filing: 18.05.2021
(51) Int. Cl.: A61K 35/644, A61K 36/47, A61K 31/13, A01N 61/02, A61P 31/04, A61P 31/10, A61P 31/12

(54) **NATURAL ANTIBACTERIAL AND ANTIVIRAL BIOLOGICAL COMPOSITIONS, COMPOUNDS, METHOD FOR OBTAINING SAME, AND USE THEREOF**

(30) Priority: 19.05.2020 BR 102020009939
(71) Applicant: Beeotec Holding S/A, 240-020 Curitiba (BR)
(72) Inventor: COSTA, Francisco Eduardo De Carvalho, 37540-000 Santa Rita do Sapucaí-MG (BR); RIBEIRO, Maria Cristina Marcucci, 13024050 Campinas - SP (BR)
(74) Representative: Jacob, Reuben Ellis
(86) International application number: PCT/BR2021/050207
(87) International publication number: WO 2021/232125

(57) **Abstract**

The present invention relates to a natural antibacterial and antiviral biological composition for use in hospitals and homes, in particular a composition that includes ricinoleic acid derived from the extraction of castor oil, diethanolamine and propolis, a method for obtaining the composition, and the use thereof. The invention can be used to control various pathogenic microorganisms, such as bacteria belonging to the Staphylococcus, Pseudomonas, Enterobacter and Bacillus genera; as well as hospital-environment fungi (filamentous and yeast-like) and phytopathogenic fungi (Colletrotichum fragariae). The product can be sprayed on biotic and abiotic surfaces, and non-woven fabric can subsequently be used to remove organic matter, including microorganisms. The product can also be used to disinfect medical, dental and hospital items by immersion followed by quick rinsing.

## Description

The present invention relates to natural antibacterial and antiviral biological compositions for use in hospitals and home area, in particular, biocidal compounds, which comprise ricinoleic acid from the extraction of castor oil, diethanolamine and propolis, method for obtaining the compounds, and the use thereof. The present invention can be used to control various pathogenic microorganisms, such as bacteria belonging to the *Staphylococcus, Pseudomonas, Enterobacter* and *Bacillus* genera; as well as hospital-environment fungi (filamentous and yeast-like) and phytopathogenic fungi (*Colletrotichum fragariae*)*.* The products can be sprayed on biotic and abiotic surfaces, and non-woven fabric can subsequently be used to remove organic matter, including microorganisms. The products can also be used in the disinfection of medical, dental and hospital items, by immersion followed by quick rinsing. Due to its low toxicity, it can be used to sanitize hands and forearms, and can be used as a disinfectant soap.

In recent decades, there has been an emerging need for natural alternatives to combat pathogenic microorganisms, which minimize damage or have low environmental impact and low toxicity.

In the prior art, natural sanitizing and disinfecting compositions containing extracts of native plants are known. Such compositions require quite a long contact time to provide effective antibacterial action. In addition, many antibacterial actives, in addition to being abrasives, require hours before an effective antibacterial action is achieved.

Biocidal compositions are commonly used to disinfect and control the growth of microorganisms, but they are not effective against a wide range of spores, and their effects do not last long enough. Very few chemical agents, or combinations thereof, have timely sporicidal action.

A significant advance in the prior art would be to provide a biocide with increased efficacy at lower concentrations and toxicity, as well as effectiveness against a wider spectrum of pathogenic microorganisms.

The present invention discloses totally clear biocidal compositions resulting from the association of ricinoleic acid from the extraction of castor oil, diethanolamine and propolis in order to enhance antibacterial and antiviral activity. The final compounds of the association result in a totally clear solution with colloidal stability, since propolis is easily incorporated into the mixture of ricinoleic acid and diethanolamine (called Ricinolamide), without forming micelles that could result in a cloudy solution. Due to the detergent properties of the biocide, the dissolution of the propolis occurs, forming a clear solution, and a greater amount of propolis can be used in the formulation, depending on the use. Furthermore, the propolis dissolved in this way is not sticky to the touch.

### PRIOR ART

Currently, many products intended for use as synthetic and natural biocides are widely known by the current prior art, most of them used to control organisms that are considered harmful, such as herbicides, pesticides, insecticides and bactericides. Biocides are also used for water treatment, in the leather industry and in oil production, among many other areas.

ln the article presented in June 1961 by the Louisiana State University, it is reported that derivatives of ricinoleic acid and oleic acid were investigated for their antimicrobial activity, under ideal growth conditions, against various species of bacteria, yeasts and molds. Several derivatives of ricinoleic acid and petroselinic acid (iso-oleic) have exhibited considerable antimicrobial activity; the activity against some microorganisms was comparable to that of sorbic acid and 10-undecenoic acid, known antimicrobial agents, as indicated by the tests.

The master's dissertation at Universidade Estadual Paulista in May 2008, aimed to test the control of contaminating bacteria in fermentation vats, using esters obtained from the chemical synthesis of castor oil (Ricinus communis L.).

The master's research presented at Universidade Estadual Paulista in August 2010 focused on evaluating the efficiency of natural antimicrobials and their interaction with conventional antibiotics in controlling bacterial contamination in *cachaça* production. Among the treatments, the brown propolis extract, green propolis, ampicillin and interaction (P/A) maintained the yeast cell viability indexes at high levels and above 90%, and reduced bacterial contamination, with lower rates observed for the control treatment.

The researches prospected in the scientific literature did not even suggest as future considerations or perspectives the development of a composition comprising combining the components of the present invention with ricinoleic acid, diethanolamine and propolis, and that this could result in a biocide with superior effects.

PI0807634 relates to biocidal compositions comprising dialkylamides, and their use as solvents or dispersants for biocides. The technical problem solved by this document is the solubilization and dispersion of biocides, preventing the formation of crystals in the solution, increasing the shelf life and reducing the risk of clogging of filters and nozzles during the application of the biocide.

US patent 9,783,435 reports methods for controlling organisms by applying biocide compositions comprising castor oil and/or derivatives having activity to kill or inhibit the proliferation of microorganisms, wherein the composition comprises ricinoleic acid, castor oil and diethanolamine. In another aspect, it presents a method for controlling viruses comprising a step of contacting a medium comprising viruses and a biocide composition comprising from 40% to 100% by volume of ricinoleic acid and from 0% to 60% by volume of castor oil (castor oil). This document further discloses a method to prevent corrosion on surfaces and inhibit the action of sulfate reducing bacteria and bacteria responsible for biocorrosion, to control microorganisms or barnacles, more preferably to control BRS at oil drilling sites or to control the growth of barnacles on parts of ships.

Additionally, current sanitizing and disinfecting products are also very corrosive, damaging the surfaces on which they are used.

Treatments with a biocide alone, in some cases, are not enough to contain contamination. However, instead of individual use, the combination of antimicrobials may present better results in disinfection and control of contaminants.

It is known that existing products in the prior art are chemical products, of high toxicity, of difficult biodegradability, causing residues of chemical compounds, harmful to the environment and to human beings.

Thus, the development of new technologies and products to combat contaminants, as presented in the present invention, will be of great help in the production of sanitizing detergents for domestic, agricultural use (cleaning machinery, cleaning floors and surfaces, cleaning animals), hospital use (both in sanitization and disinfection).

ln this way, the design of the present invention, resulting from the association of ricinoleic acid from the extraction of castor oil, diethanolamine and propolis, is based on a formula that improved existing technologies and brought solutions to the problem of achieving high antibacterial efficacy with synergistic activity, which is not achieved individually by each of the components.

Propolis is a product made by bees from plant sources, such as sprouts and exudates. The word propolis comes from the Greek and means pro, in defense of and polis the city. Bees collect this resinous and/or balsamic material, consisting of secondary metabolites produced by plants. Secondary metabolites are substances that plants produce to defend themselves against predators, viruses, bacteria and fungi. Therefore, if bees use this material for the production of propolis, it would be expected that it would have the same defense properties. This fact has been proven over almost one hundred years of research in different parts of the world with different types of propolis, evidencing its effectiveness against microorganisms, tumors and strengthening the immune system (Marcucci, 1995). Propolis has proven antimicrobial activity, being effective in fighting bacteria, fungi and viruses. There are numerous reports informing its bactericidal power in Gram positive and negative bacteria (Miorin et al., 2003; Veiga et al., 2017). Brazilian propolis extracts were studied against poliovirus type 1 (PV1) of poliomyelitis, influenza (influenza), HIV, H1N1, viral hepatitis, herpes virus type 1 and type 2, viruses involved in dengue hemorrhagic fever and in coronaviruses of animal origin. (Urushisaki et al., 2011; Takeshita et al., 2013; Soroy et al., 2014; Peng et al., 2016; Yildirim et al., 2016; Mounieb et al., 2017; Governa et al., 2019; Münstedt, 2019).

Ricinoleic acid is the main component of the oil extracted from castor bean seeds (Ricinus communis L.) having in its composition fatty acids in addition to flavonoids and other compounds (Upaisini et al., 2003).

The essential oil of Ricinus communis L. leaves was analyzed by gas chromatography coupled to a mass detector (GC-MS) and bioassays were performed. Five oil constituents were identified. The antimicrobial activity was investigated, evaluating its effectiveness against twelve bacteria and four species of fungi, namely: *Staphylococcus* aureus 1327, *Staphylococcus* epidermidis, Micrococcus luteus, Enterococcus faecalis, *Enterobacter* cloacae, *Staphylococcus* aureus 25923, *Bacillus* subtilis, *Bacillus* cereus, *Pseudomonas* aeruginosa 27853, Klebsiella pneumoniae WHO24, Escherichia coli 25922 and the fungi: Botrytis cinerea, Fusarium solani, Penicillium digitatum and Aspergillus niger. The essential oil showed strong antimicrobial activity against all microorganisms tested with greater sensitivity to *Bacillus* subtilis, *Staphylococcus* aureus and *Enterobacter* cloacae. The cytotoxic and apoptotic effects of the essential oil on HeLa cell lines were examined by the MTT assay. The cytotoxicity of the oil was quite strong with IC50 values below 2.63 mg/mL (Zarai et al., 2002). In general, the following activities have been described for castor oil: antidiabetic, antibacterial and antifungal, insecticidal, antioxidant, anti-inflammatory, analgesic, antitumor, antiasthmatic (Rana et al., 2012) laxative (Cruz e Silva, 2017) among others.

The insecticidal and nematicicidal properties of the oil have been proven (Jaramillo, 2015; Sanaguano, 2018) as well as its use in animal feed (Furlan, 2017; Percio et al., 2019).

It was found that only with the use of compounds resulting from the association of castor oil and diethanolamine or only with the use of propolis, the biocidal effect is not as efficient as the proposed invention.

It was found that the combination of ricinoleic acid from the extraction of castor oil, diethanolamine and propolis, in certain weight/concentration ratios, generates synergistic effects when used to control phytopathogenic fungi, especially *Colletrotichum fragariae,* where the addition of propolis produced 33% higher inhibition halos compared to pure propolis and ricinoleic acid ester alone. That is, the combined components result in improved biocidal properties compared to the individual or isolated properties and performance of each component, indicating a synergistic effect.

Still referring to this embodiment, the propolis used in the present invention is called BRPX, rich in prenylated compounds.

### SUMMARY OF THE INVENTION

The present invention comprises biocidal compositions arranged in liquid and clear form, obtained through a specific obtaining method and a general composition based on defined proportions of ricinoleic acid from the extraction of castor oil and propolis.

ln accordance with an aspect of the invention, it is provided a biocidal composition comprising:
(a) 1.4 to 5.0% by weight of ricinoleic acid selected from the group consisting of fatty acids;
(b) 0.5 to 2.0% by weight of diethanolamine selected from the group consisting of amine dialcohol;
(c) 0.1 to 3%, propolis selected from the group consisting of prenylated compounds;

Exemplary embodimentof the invention - Composition with bactericidal and antiviral effect (Table 1).

**Table 1 - Components of the compound developed in this exemplary embodiment.**

| Component | | Component Action | Concentration (%) |
|---|---|---|---|
| Ricinolamide (ricinoleic acid diethanolamine) | + | *Staphylococcus, Pseudomonas*, *Enterobacter* and *Bacillus*; hospital-environment fungi (filamentous and yeast-like) and phytopathogenic fungi (*Colletrotichum fragariae*) | 7.0 |
| Propolis* | | Antibacterial (*S.aureus, E.coli*, *Pseudomonas*) | 3.0 |
| | | Antifungal (*C.albicans*) | |
| | | Antiviral (poliovirus type 1, influenza, HIV, H1N1, viral hepatitis, herpes virus types 1 and 2, viruses involved in dengue hemorrhagic fever and coronaviruses of animal origin) | |

| | | | |
|---|---|---|---|
| * Dry ethanolic extract. | | | |

The present embodiment of the invention presented the following technical characteristics, in addition to having a clear and translucent appearance, with a color ranging from amber to water white, when in the proportion of 7% of ricinolamine to 0.1% of propolis.

**Table 2 - Physicochemical characteristics of the compound in the formulation in the proportion of 7% ricinolamine to 0.1% propolis.**

| Physical-chemical test | Mean ± SD |
|---|---|
| Ricinolamide concentration | 2.0 a 7.0% |
| Propolis concentration | 0.1 a 3.0% |
| Electrical conductivity (µS/cm) | 926.00 ± 56.72 |
| Color (mm Pfund) | 8.539 ± 0.192 (water white) |
| Density (g/cm³) | 1.0100 ± 0.0027 |
| Fluidity (viscosity) | Runoff time: 28 s |
| | Value (0.49*(t-35)) CsT: - 3.43 |
| | CentiPoise: - 3,44 |
| pH | 8.10 ± 0.06 |

Microscopic appearance: the sizes of the dispersed antigenic phase droplets are less than 10 micrometers and poorly dispersed.

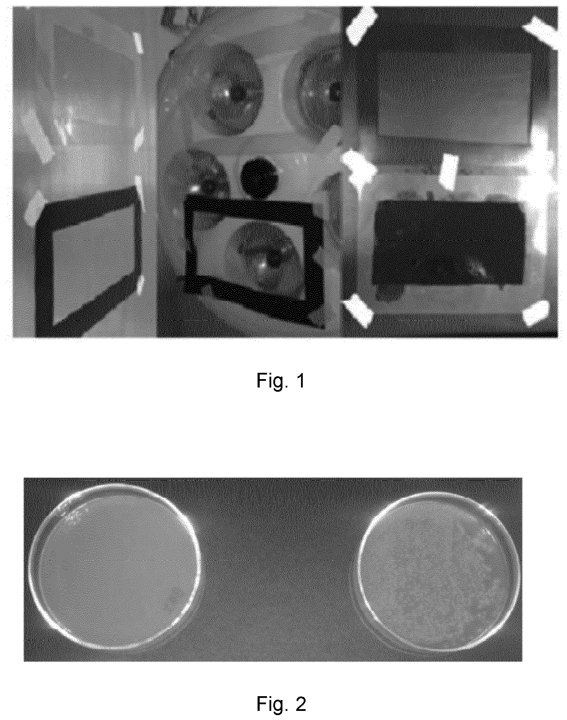

Hospital infection is a serious public health problem, as it is responsible for the increase in the mortality rate of patients that occurs since the period of hospitalization and the increase in assistance costs. Additionally, a study was carried out to identify among three disinfectant products (70% isopropyl alcohol, biological compound of the present invention and sodium hypochlorite) which is the best to reduce the count of microorganisms on surfaces of a surgical center of a philanthropic hospital in the south of Minas Gerais, Brazil. The study period was carried out in 2019. The collection points chosen were the surgical table, the surgical lighting and the support cart, where the instruments used by the medical team are located. In order to delimit the area to be tested, sterile 25cm² templates were used (Fig. 1). The collection points were characterized from left to right: surgical table, surgical lighting and support table. The templates were produced in polymer to standardize the areas to be sampled.

Pre-disinfection and post-disinfection collections were made at each point, in different areas of the same surface, using three disinfectant products, 70% isopropyl alcohol, 0.5% sodium hypochlorite and the biocidal composition of the present invention. The disinfectants were applied as a spray to cover the delimited area, waited 5 minutes, passed a sterile gauze in a single direction, and allowed to dry the surface. Two visits to the hospital were carried out for each disinfectant, with plating in triplicate. Sterile swabs were used for collection, passing it in the delimited area in three directions at an angle of 30°. The head of the swab shaft was stored in sterile tubes, previously identified with the point number and the experimental treatment (pre or post-disinfection). Transport was carried out in a styrofoam box with rigid reusable ice. The medium used (USP, 2013) were Brain Heart Infusion Agar (BHI) for the growth of heterotrophic bacteria and Potato Dextrose Agar (PDA) for molds and yeasts. For microbiological analysis, each tube received 9 mL of sterile saline solution and was shaken vigorously in circular motions using a tube shaker at maximum speed for 30 s, before surface plating and incubation (APHA, 1998). In surface plating for molds and yeast analysis, 0.1 mL of the sample solution was pipetted onto the surface of the PDA agar and spread with the aid of a previously sterile Drigalski loop with flammable alcohol and flamed in a Bunsen burner. After five minutes, the plates were incubated in a bacteriological incubator at 28 °C. For the cultivation of heterotrophic bacteria, in the pour-plate technique, 1 mL of saline solution was pipetted after shaking (cell suspension) in sterile Petri dishes and then BHI Agar was added, still liquid, however previously cooled, and incubated in a bacteriological incubator at 36 °C. The plates were kept in the incubators for a minimum of 72 hours before counting the Colony Forming Units (CFU).

Analysis of microbial count results was conducted using R software with analysis of variance (P < 0.05). Data were evaluated separately, but always between two different groups, one being the control (pre-disinfection) and the other being the post-disinfection, in order to verify that treatment differs significantly from the control. The variables were submitted to descriptive analysis expressed through the mean and standard deviation. After the descriptive analysis, the T-Student test was performed to compare means with independent populations, comparing the control group with the samples (STEEL and TORRIE, 1980).

In the quantitative analysis, the biocidal compound, object of the invention, showed the greatest effectiveness (Figure 2). The product showed satisfactory effectiveness as a disinfectant against bacteria (Table 3) as well as against fungi (Table 4), as it decontaminated most of the microorganisms present on the different surfaces tested, with an efficiency equal to or greater than the other two products (Table 5). A fact that should also be considered is its characteristic of being derived from castor beans, meeting one of the principles of green chemistry (LEONARDO et al. 2003). Other important points are the fact that the natural biological biocide of the present invention does not harm surfaces and has a short action time. Bacteria were identified by classical methods and test on modified Rugai medium. Bacteria belonging to the genera *Enterobacter, Pseudomonas* and *Staphylococcus* stood out. Most of the fungal isolates were yeast-like fungi. The presence of Aspergillus niger and Aspergillus flavus was observed. Ethyl alcohol at 70% and isopropyl alcohol at 70% are the main disinfectants used in Brazil because they have the lowest cost compared to the others. Alcohol is a bactericide capable of eliminating most of the microorganisms present, with the exception of bacterial spores, which are the most resistant state of Gram positive bacteria. One of the disadvantages of using this product is the risk of acting as a solvent when applied on polymers, as it represents a risk of damage to equipment. Another common cleaning method uses chlorine-based disinfectants. Disinfection cycles lasting 5 to 20 minutes are performed. The risk lies in the fact that chlorine-based products are highly corrosive to metal surfaces and dry out polymers.

In (Fig. 2) the microbial growth collected on the surface of the surgical lighting after application of the biocide of the invention (left) and without application (right) is shown. Note that each opaque spot on the surface of the BHI agar medium represents a CFU.

**Table 3 - Quantification of colonies in BHI medium using the biocide of the invention.**

| Collect point | Date | Plate | CFUs/mL |
|---|---|---|---|
| Contaminated surgical table | 02/Jan/2019 | BHI 1 | 10 |
| | 02/Jan/2019 | BHI 2 | 0 |
| | 02/Jan/2019 | BHI 3 | 20 |
| Disinfected surgical table | 02/Jan/2019 | BHI 1 | 10 |
| | 02/Jan/2019 | BHI 2 | 0 |
| | 02/Jan/2019 | BHI 3 | 0 |
| Contamined surgical lighting | 02/Jan/2019 | BHI 1 | 1340 |
| | 02/Jan/2019 | BHI 2 | 4880 |
| | 02/Jan/2019 | BHI 3 | 3320 |
| Desinfected surgical lighting | 02/Jan/2019 | BHI 1 | 0 |
| | 02/Jan/2019 | BHI 2 | 0 |
| | 02/Jan/2019 | BHI 3 | 0 |
| Contaminated support cart | 02/Jan/2019 | BHI 1 | 0 |
| | 02/Jan/2019 | BHI 2 | 20 |
| | 02/Jan/2019 | BHI 3 | 30 |
| Disinfected support cart | 02/Jan/2019 | BHI 1 | 0 |
| | 02/Jan/2019 | BHI 2 | 0 |
| | 02/Jan/2019 | BHI 3 | 0 |

**Table 4 - Quantification of fungal colonies in BDA medium using the biocide of the invention.**

| Collect point | Date | Plate | CFUs/mL |
|---|---|---|---|
| Contaminated surgical table | 04/Oct/2019 | BDA 1 | 0 |
| | 04/0ct/2019 | BDA 2 | 0 |
| | 04/Oct/2019 | BDA 3 | 0 |
| Disinfected surgical table | 04/0ct/2019 | BDA 1 | 0 |
| | 04/Oct/2019 | BDA 2 | 0 |
| | 04/0ct/2019 | BDA 3 | 0 |
| Contamined surgical lighting | 04/Oct/2019 | BDA 1 | 1000 |
| | 04/0ct/2019 | BDA 2 | 2420 |
| | 04/Oct/2019 | BDA 3 | 1750 |
| Desinfected surgical lighting | 04/0ct/2019 | BDA 1 | 0 |
| | 04/Oct/2019 | BDA 2 | 0 |
| | 04/0ct/2019 | BDA 3 | 0 |
| Contaminated support cart | 04/Oct/2019 | BDA 1 | 120 |
| | 04/0ct/2019 | BDA 2 | 60 |
| | 04/Oct/2019 | BDA 3 | 50 |
| Disinfected support cart | 04/0ct/2019 | BDA 1 | 0 |
| | 04/Oct/2019 | BDA 2 | 0 |
| | 04/0ct/2019 | BDA 3 | 0 |

**Table 5 - Efficiency of the different disinfectants tested.**

| Disinfectant | Reduction of counts microbial (%) |
|---|---|
| 70% isopropanol | 87 a |
| Biocide of the invention | 83 a |
| 0.5% sodium hypochlorite | 61 b |

| | |
|---|---|
| Values followed by the same letter do not differ significantly (p<0.05). | |

### DETAILED DESCRIPTION OF THE INVENTION

The present invention provides a totally clear natural biocidal composition resulting from the association of ricinoleic acid from the extraction of castor oil, diethanolamine and propolis in order to enhance antibacterial and antiviral activity. The compound resulting from the association is completely clear, as the propolis is easily incorporated into the mixture of ricinoleic acid and diethanolamine, without forming micelles that could result in a cloudy solution. Due to the detergent properties of the biocide, the dissolution of the propolis occurs, forming a clear solution, and a greater amount of propolis can be used in the formulation, depending on the use.

The composition preferably comprises 1.4 to 5.0%, more preferably 2.0 to 4.0%, by weight of ricinoleic acid. Ricinoleic acid is added to diethanolamine. The reaction of ricinoleic acid (A) with diethanolamine (B), produces an amide (C), called ricinolamide. In this reaction, the formation of the amide and the loss of water occur.

Below is the structure of the mixture of ricinoleic acid and diethanolamine, as well as the product Ricinolamide formed:

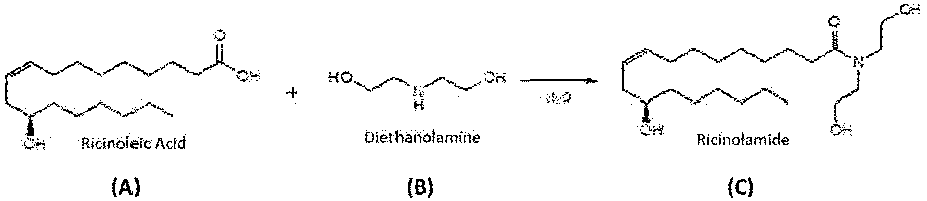

Chemical reaction of ricinoleic acid (A) with diethanolamine (B) forming the amide called ricinolamide (C).

The ricinoleic acid is preferably selected from commercial batches. The compound comprises from 1.4 to 5.0%, more preferably 2.0 to 4.0%, of ricinoleic acid.

Diethanolamine is preferably selected from commercial batches. The compound comprises from 0.5 to 2.0%, more preferably from 1.0 to 1.5%, of diethanolamine.

Another aspect of the present invention relates to a method for obtaining an amide compound with antibacterial and antiviral effect, comprising the steps of:
(a) 25% diethanolamine (A) and 75% ricinoleic acid (B) are added to a reactor with mechanical agitation;
(b) The two components (A) + (B) are mixed for 2 hours, monitoring the temperature, as it is an exothermic reaction;
(c) The crude product is diluted in warm water, from 40° C to 60° C, until its complete dissolution. The reaction is carried out without a catalyst, not exceeding 60° C, in a controlled environment at 20° C;
(d) Final dilution is done in water, resulting in a 50% solution.

If we consider the equimolar reaction (1 mol of ricinoleic acid reacts with 1 mol of diethanolamine forming 1 mol of the amide and 1 mol of water) we have: 75% ricinoleic acid (RA) at 95% purity reacting with 25% diethanolamine (DIET). As the molecular weight of RA is 298.46 g/mol and of DIET 105.14 g/mol and correcting the mass of RA (discounting impurities) we have: 71.25 g of RA which is 0.24 mol and 25.0 g of DIET which is 0.24 mol. Therefore the compounds react in equimolar amounts to form 0.24 mol of the amide (C) plus 0.24 mol of water. We have the results shown in Table 6.

**Table 6 - Stoichiometric calculation of the Ricinolamide formation reaction.**

| Reagents | | Products | | |
|---|---|---|---|---|
| Name | Weight (g) | Name | Weight (g) | Yield (%) |
| RA | 71.25 | Amide | 92.54 | 95.5 |
| DIET | 25.00 | Water | 4.32 | |
| Impurities | 3.75 | Impurities | 3.14 | |
| Total | 100.0 | Total | 100.00 | |

Therefore, the yield of the reaction of ricinoleic acid with diethanolamine, forming the amide, is 95.5%.

### 1) Chemical characteristics of propolis:

The propolis used in the present invention is called BRPX, rich in prenylated compounds, as described below:

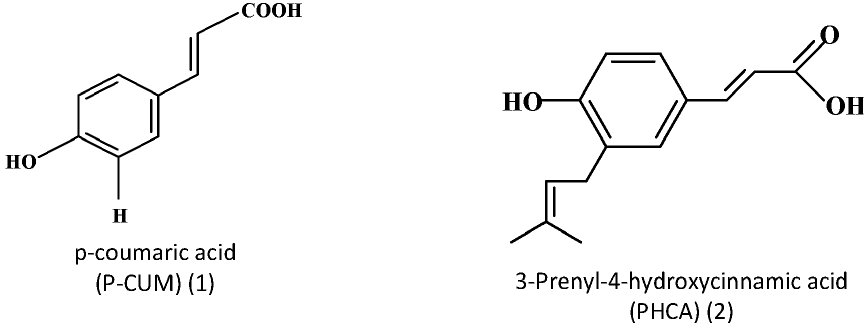

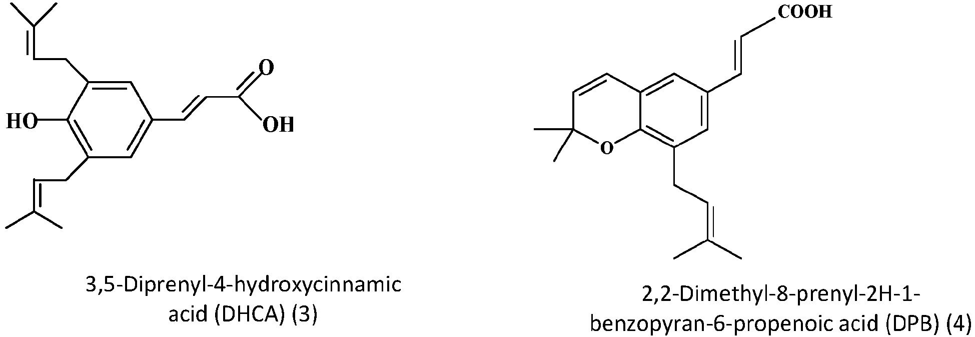

Main markers found in BRPX propolis, with DHCA (Artepillin-C) being the majority.

Below are the quality parameters referring to the sample used in the present study: total phenolic compounds: 9.0 to 10.0%, total flavonoids between 2.5 and 3.5%, insoluble residues between 35.0 and 45.0%, ethanol soluble solids between 45.0 and 50.0%, moisture (loss on desiccation) between 4.0 and 5.0% and wax content between 9.0 and 10.0%.

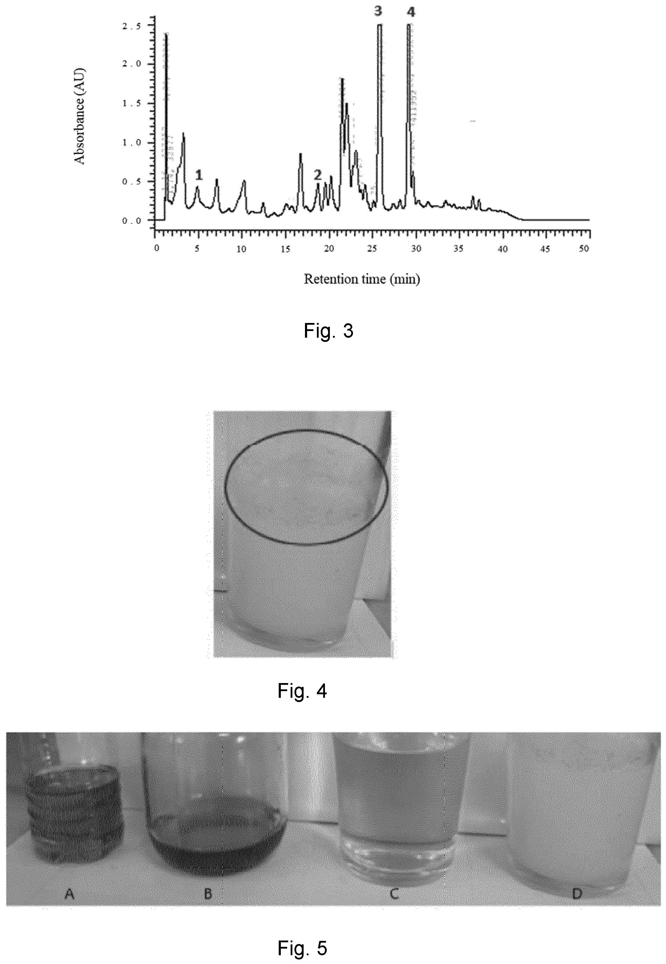

In Fig. 3, analysis of the same sample by high performance liquid chromatography (HPLC) is shown.

The quantitative analysis of these markers showed levels of: p-coumaric acid (1) between 1.5 and 2.0%; PHCA (2) between 0.3 to 0.5%; Artepillin-C (3) between 3.0 and 5.0; DPB (4) between 0.3 to 5.0%. The antioxidant activity, evaluated by the DPPH radical decolorization method (Veiga et al., 2017) was between 5.0 and 15.0 µg/mL.

Propolis has great difficulty in solubilizing in water, forming micelles and clouding the solution. Some companies have launched propolis-based products on the market, stating on the label that they are aqueous extracts.

As the main characteristic of propolis is that it is a resinous substance, whose bioactive compounds are practically insoluble in water, except for p-coumaric acid and caffeoylquinic acids, it is not to be expected that clear solutions will be present. Due to the nonpolar characteristic of the main bioactive compounds, these in contact with water form micellar structures, as shown in Fig. 4. One way to solubilize propolis to form a clear solution is to add a base, such as sodium hydroxide, to water.

The acids in propolis (major compounds) react with the base, forming salt and water, but the final solution, although clear, has basic characteristics, with a pH between 8.0 and 9.0, not being suitable for human consumption.

Figure 4 - The circled area corresponds to a "cream" formed on the walls of the container, indicating the incompatibility of propolis with water.

The propolis should preferably be present at 0.1 to 3.0%, more preferably from 0.2 to 1.0%, by weight of the composition.

The biocidal compound, object of this invention, can be used to develop various sanitizing detergent products for domestic, agricultural (cleaning machinery, floors, surfaces and animals), hospital (sanitization and disinfection) use. Examples include the presentation of products in the form of lotions, gels, sprays, shampoos, etc.

Antibacterial and antiviral benefits are provided by the fast acting compound of the invention and therefore it is especially suitable for incorporation into biocidal products.

It is preferred that the compound of the invention be formulated to have a pH of 7.5 to 8.5, preferably between 8 and 8.3, wherein the effectiveness of the synergistic interaction between ricinoleic acid, diethanolamine and propolis is seen as maximum.

The biocidal compound can also be used to clean and disinfect topical areas such as wounds and therefore can be formulated as a gel or ointment.

### Formulations of the Invention

Control ricinolamide at 10% (aqueous solution): 10 g of the product in 100mL (final volume in a volumetric flask, the meniscus is adjusted with water). Amount of the active: 9.55%.

Control propolis at 3% (solution in ethanol or DMSO): 3 g of the product in 100mL (final volume in a volumetric flask, the meniscus is adjusted with ethanol or DMSO). Amount of the actives: p-coumaric acid (1) from 0.045 to 0.06% (450 to 600 µg/mL); 3-prenyl-4-hydroxycinnamic acid (2) from 0.009 to 0.015% (90 to 150 µg/mL); 3,5-diprenyl-4-hydroxycinnamic acid (3) from 0.09 to 0.15% (900 to 1500 µg/mL); 2,2-dimethyl-8-prenyl-2H-1-benzopyran-6-propenoic acid (4) between 0.009 to 0.15% (90 to 1500 µg/mL).

Biocidal Composition 1: 0.2% propolis and 5% ricinolamide: 5g of ricinolamide and 0.2g of propolis. Amount of the active ricinolamide: 4.8%. Amount of actives: p-coumaric acid (1) from 0.003 to 0.004% (30 to 40 µg/mL); 3-prenyl-4-hydroxycinnamic acid (2) from 0.0006 to 0.001% (6 to 10 µg/mL); 3,5-diprenyl-4-hydroxycinnamic acid (3) between 0.006 to 0.01% (60 to 100 µg/mL); 2,2-dimethyl-8-prenyl-2H-1-benzopyran-6-propenoic acid (4) between 0.0006 to 0.01 % (6 to 100 µg/mL).

Biocidal Composition 2: 0.5% of propolis and 3% of ricinolamide: 3g of ricinolamide and 0.5g of propolis. Amount of the active ricinolamide: 2.86%. Amount of actives: p-coumaric acid (1) from 0.0075 to 0.01% (75 to 100 µg/mL); 3-prenyl-4-hydroxycinnamic acid (2) from 0.0015 to 0.0025% (15 to 25 µg/mL); 3,5-diprenyl-4-hydroxycinnamic acid (3) between 0.015 to 0.025% (150 to 250 µg/mL); 2,2-dimethyl-8-prenyl-2H-1-benzopyran-6-propenoic acid (4) between 0.0015% to 0.025% (15 to 250 µg/mL).

Biocidal Composition 3: 0.1% propolis and 7% ricinolamide: 7g of ricinolamide and 0.1g of propolis. Amount of the active ricinolamide: 6.68%. Amount of actives: p-coumaric acid (1) from 0.0015 to 0.002% (15 to 20 µg/mL); 3-prenyl-4-hydroxycinnamic acid (2) from 0.0003 to 0.0005% (3 to 5 µg/mL); 3,5-diprenyl-4-hydroxycinnamic acid (3) between 0.003 to 0.005% (30 to 50 µg/mL); 2,2-dimethyl-8-prenyl-2H-1-benzopyran-6-propenoic acid (4) between 0.0003 to 0.005% (3 to 50 µg/mL).

Biocidal Composition 4: 3% propolis and 2% ricinolamide: 2g of ricinolamide and 3g of propolis. Amount of the active ricinolamide: 1.90%. Amount of actives: p-coumaric acid (1) from 0.045 to 0.06% (450 to 600 µg/mL); 3-prenyl-4-hydroxycinnamic acid (2) from 0.009 to 0.015% (90 to 150 µg/mL); 3,5-diprenyl-4-hydroxycinnamic acid (3) from 0.09 to 0.15% (900 to 1500 µg/mL); 2,2-dimethyl-8-prenyl-2H-1-benzopyran-6-propenoic acid (4) between 0.009 to 0.15% (90 to 1500 µg/mL).

Obtaining method:
a) Weigh 0.1 to 3.0g of propolis in a previously weighed 100 mL beaker;
b) Weigh 2.0 to 7.0g of ricinolamide in a previously weighed 50 mL beaker;
c) Heat the ricinolamide to 50 °C;
d) Pour the ricinolamide into the 100 mL beaker where the propolis was weighed;
e) Mix gently with the aid of a glass stick;
f) Place water at 40 °C in the 50 mL beaker until it is almost full;
g) After the ricinolamide is fully mixed with the propolis, add the water contained in the 50 mL beaker little by little to this mixture, gently shaking;
h) Transfer the mixture to a 100 mL flask;
i) Add water to the 50 mL beaker and continue pouring into the 100 mL flask with the aid of a Pasteur pipette;
j) Hit the meniscus with water;
k) Shake gently and transfer to a labeled flask.

### Molecular structures of compounds

### Compound 1: C₃₁H₄₉NO₆ - Exact mass: 531.36 g/mol

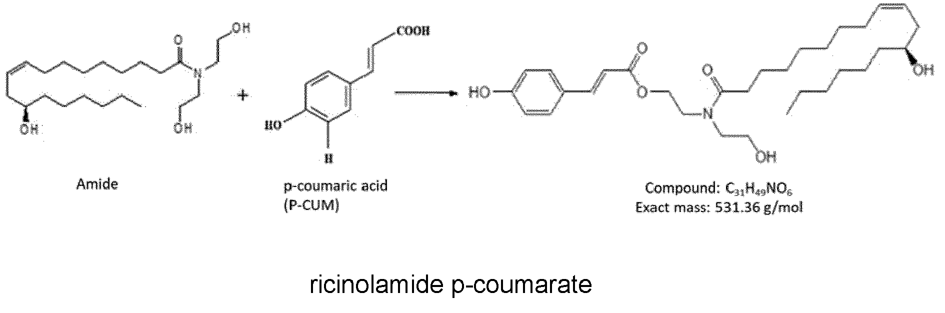

### Compound 2: C₃₆H₅₇NO₆ - Exact mass: 599.42 g/mol

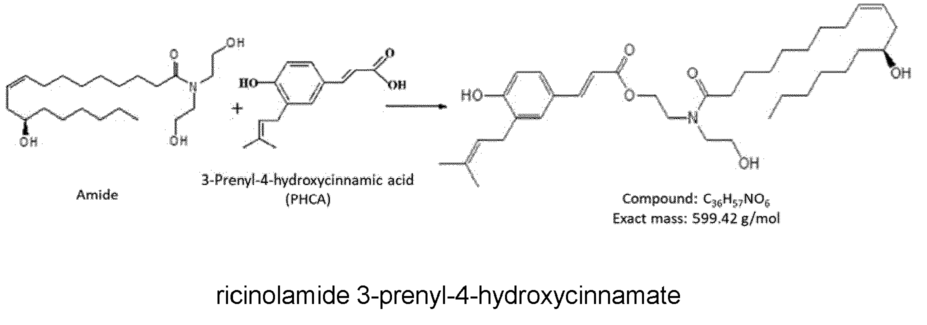

### Compound 3: C₄₁H₆₅NO₆ - Exact mass: 667.48 g/mol

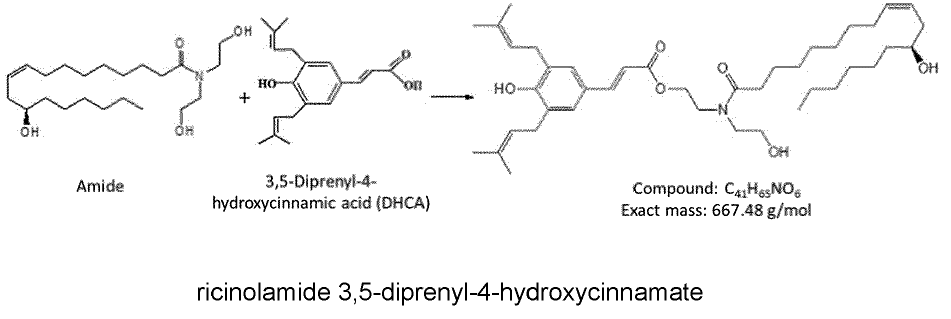

### Compound 4: C₄₁H₆₅NO₆ - Exact mass: 667.48 g/mol

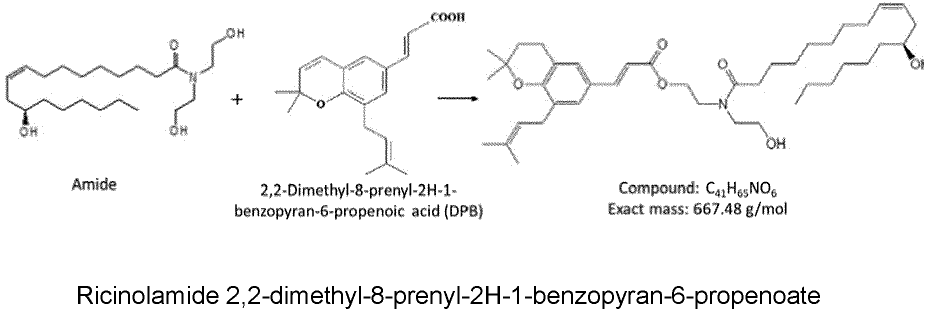

Ricinolamide (Fig. 5/A) mixes easily with the dry ethanolic extract of propolis, called "soft extract". It forms a solution with oily characteristics (Fig. 5/B). The process of preparing the mixture must be done with very gentle agitation so as not to incorporate air bubbles.

Fig. 5 - Containers showing the ricinolamide (Fig. 5/A), the ricinolamide with the soft extract of propolis without alcohol (Fig. 5/B), the aqueous solution of the ricinolamide with propolis (Fig. 5/C) and the alcoholic tincture of propolis in water (Fig. 5/D).

lt was found, quite unexpectedly, that the mixture of ricinolamide with the soft extract of propolis without alcohol and then diluted in water, forms a clear solution, as seen in Fig. 5/C unlike the alcoholic extract of propolis in contact with water, Fig. 5/D

Obtaining this clear solution Fig. 5/C is characterized by the physical-chemical parameters mentioned in Table 2.

## Claims

1. NATURAL ANTIBACTERIAL AND ANTIVIRAL BIOCIDAL COMPOSITION, wherein it comprises: ricinoleic acid selected from the group consisting of fatty acids, diethanolamine selected from the group consisting of amine dialcohol and propolis selected from the group consisting of prenylated compounds.

2. COMPOSITION, according to claim 1, wherein it is obtained by reacting ricinoleic acid, preferably from 2.0 to 4.0% by weight, diethanolamine preferably from 1.0 to 1.5% by weight and propolis extract, preferably from 0 .2 to 1.0% by weight.

3. COMPOSITION, according to claims 1 and 2, wherein the propolis used to prepare the extract is of the BRPX type.

4. COMPOSITION, according to claims 1 to 3, wherein the propolis extract comprises one or more prenylated compounds, such as pcoumaric acid and its derivatives.

5. COMPOSITION, according to claims 1 to 4, wherein it is presented in the form of gel, ointment, lotions and liquid solution.

6. OBTAINING METHOD, wherein it comprises the following steps:
(a) weigh 0.1 to 0.3 g of propolis in a previously weighed 100 mL beaker;
(b) weigh 2.0 to 7.0 g of ricinolamide in a previously weighed 50 mL beaker;
(c) heat the ricinolamine to 50 °C;
(d) pour the ricinolamide into the 100 mL beaker where the propolis was weighed;
(e) mix gently with the aid of a glass stick;
(f) place water at 40 °C in the 50 mL beaker until it is almost full;
(g) after the ricinolamide is fully mixed with the propolis, add the water contained in the 50 mL beaker little by little to this mixture, gently shaking;
(h) transfer the mixture to a 100 mL flask;
(i) add water to the 50 mL beaker and continue pouring it into the 100 mL flask with the aid of a Pasteur pipette;
(j) hit the meniscus with water;
(k) gently shake and transfer to a labeled flask.

7. COMPOUND, according to any of the preceding claims, wherein it is ricinolamide p-coumarate and has the structural formula

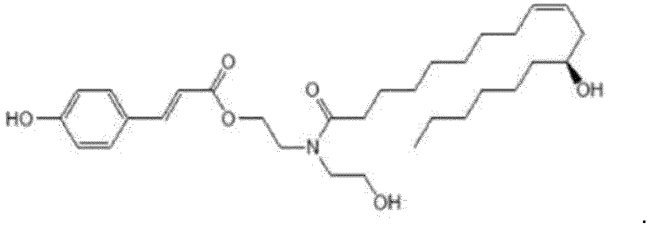

8. COMPOUND, according to any of the preceding claims, wherein it is ricinolamide 3-prenyl-4-hydroxycinnamate and has the structural formula

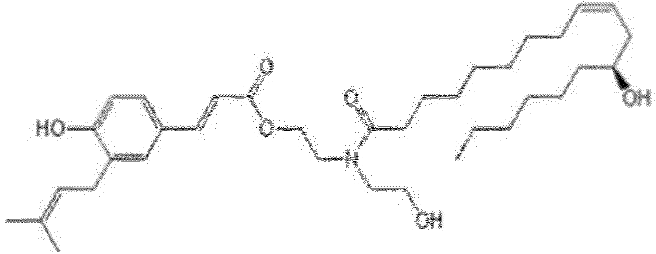

9. COMPOUND, according to any of the preceding claims, wherein it is ricinolamide 3,5-diprenyl-4-hydroxycinnamate and has the structural formula

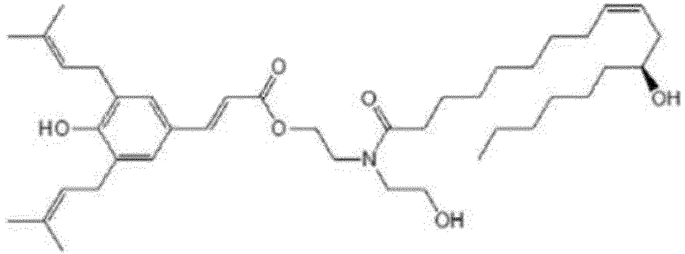

10. COMPOUND, according to any of the preceding claims, wherein it is ricinolamide 2,2-dimethyl-8-prenyl-2H-1-benzopyran-6-propenoate and has the structural formula

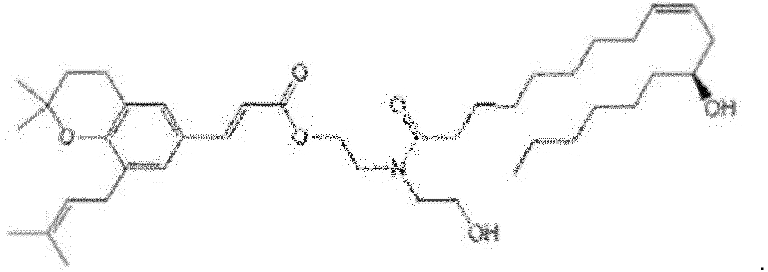

11. COMPOUND, according to claims 7 to 10, wherein it has the following physicochemical properties (a) concentration of ricinolamide 2.0 to 7.0%; (b) propolis concentration 0.1 to 3.0%; (c) electrical conductivity 926.00 ± 56.72 µS/cm; (d) Color 8.539 ± 0.192 mm Pfund; (e) Density 1.0100 ± 0.0027 g/cm³ ; (f) Fluidity (viscosity) - Runoff time: 28 s Value (0.49*(t-35)) CsT: 3.43 CentiPoise: 3.44; (g) pH of 7.5 to 8.5, preferably pH between 8 and 8.3.

12. COMPOUND, according to claims 7 to 10, wherein it is for disinfecting *Staphylococcus, Pseudomonas, Enterobacter* and *Bacillus* microorganisms; hospital-environment fungi (filamentous and yeast), phytopathogenic fungi (*Colletrotichum fragariae*)*, C.albicans,* poliovirus type 1 of poliomyelitis, influenza, HIV, H1N1, viral hepatitis, herpes virus types 1 and 2, dengue hemorrhagic virus and coronavirus of animal origin.

13. USE of the compound as defined by claims 7 to 10, wherein it is for preparing a biocide to eliminate pathogenic microorganisms.
